# EUROPEAN PATENT APPLICATION

(11) **EP 4 023 412 A1**
(43) Date of publication of application: **06.07.2022**
(21) Application number: 20859108.1
(22) Date of filing: 03.08.2020
(51) Int. Cl.: B29C 45/37, B81B 1/00, G01N 37/00, C12M 1/00

(54) **MICROCHIP**

(30) Priority: 30.08.2019 JP 2019158358
(71) Applicant: Ushio Denki Kabushiki Kaisha, Tokyo 100-8150 (JP)
(72) Inventor: SHIMONAKA, Masatoshi, Tokyo 100-8150 (JP)
(74) Representative: Tomerius, Isabel
(86) International application number: PCT/JP2020/029651
(87) International publication number: WO 2021/039296

(57) **Abstract**

Providing a microchip that effectively suppresses the peeling of a bonded substrate that has been bonded together and that is unlikely to leak liquid. A microchip includes: a bonded substrate that includes a plurality of substrates, each of the substrates having a main surface and a side surface, the main surfaces being bonded each other; and a microchannel located inside the bonded substrate. The substrates include a first substrate with a large thickness and a remaining substrate with a smaller thickness than the first substrate. At least the first substrate has a side surface having a gradient and extending to an outermost side of the bonded substrate when one end of the bonded substrate is viewed from a direction parallel to the main surface.

## Description

### TECHNICAL FIELD

The present invention relates to a microchip having a microchannel inside a bonded substrate that includes a plurality of substrates having a main surface and a side surface, the main surfaces being bonded each other.

### BACKGROUND ART

Conventionally, culturing cells or tissues has been performed using a culture dish or a culture plate on which a culture medium such as agar is applied. Since cells or tissues are cultured, observed or analyzed in a two-dimensional (planar) environment using the culture dish or the culture plate, it is unable to reproduce extracellular microenvironment. Hence, in recentyears, a microchip (also referred to as a biochip) having a microchannel capable of achieving a three-dimensional (stereoscopic) environment for culturing cells or tissue has been proposed.

Patent Document 1 described below discloses, as an example of the microchip, the formation of a bonded substrate having a hollow-shaped microchannel by bonding a main surface of a substrate having a groove formed thereon with a main surface of a substrate with a small thickness such as resin film, and the use of the bonded substrate for a microchip.

Patent Document 2 described below discloses, in the microchip using a bonded substrate, a non-bonded surface of resin film is provided in an area where a jig for positioning the bonded substrate is in contact with, in order to address the problem of the resin film peeling off when the jig for positioning the bonded substrate, which are in contact with the side surface of the bonded substrate, happens to be in contact with the resin film constituting the bonded substrate.

### CITATION LIST

### PATENT DOCUMENT

Patent Document 1: JP-A-2018-047614 Patent Document 2: International Publication WO2009/101845

### SUMMARY OF INVENTION

### Technical Problem

For microchips using bonded substrates, the microchips are usually handled in a microchip carrier to avoid touching the microchips themselves. However, there are occasionally cases in which the side surface of a microchip itself must be gripped by operator's fingers. For example, when a microchip is observed with an optical microscope, it is necessary to retrieve the microchip from the microchip carrier and grip the retrieved microchip, namely the side surfaces of the bonded substrate, with the operator's fingers. Repeatedly gripping the side surfaces of the bonded substrate may deform each of the substrates constituting the bonded substrate due to the repeated stress, causing the bonded substrate to peel off. Since operators are uncertain about which area of the side surfaces of the bonded substrate they will grip, measures such as providing a non-bonded surface on a specific area of the side surfaces are insufficient. In addition, excessive gripping force during gripping the microchip may also cause the substrate to peel off. If such peeling of the substrate reaches the microchannel, a problem can occur that liquid in the microchannel leaks out from the delaminated area.

Moreover, when a bonded substrate accommodated in a microchip carrier is handled, the bonded substrate is subjected to repeated stress from the substrate gripping means, such as shaking or vibration associated with the transportation of the microchip, arising a concern of liquid leakage from the microchannel caused by the peeling of the substrate.

The present invention was made in consideration of the above circumstances, and it is an object of the present invention to provide a microchip that effectively suppresses the peeling of a bonded substrate that has been bonded together and that is unlikely to leak liquid.

### Solution to the Problem

A microchip according to the present invention is a microchip having a microchannel located inside a bonded substrate composed of a plurality of substrates, each of the substrates having a main surface and a side surface, the main surfaces being bonded each other, wherein a plurality of the substrates are composed of a first substrate with a large thickness and the remaining substrate with a smaller thickness than the first substrate, and at least the first substrate has a side surface having a gradient and extending to an outermost side of the bonded substrate when one end of the bonded substrate is viewed from a direction parallel to the main surface.

The above-described "remaining substrate" means substrate(s) that the microchip is composed of other than the first substrate; the remaining substrate is not limited to one substrate, and may be two or more substrates.

Among a plurality of the substrates constituting a microchip, if one substrate is only deformed, there will be displacement on the bonding surfaces of the substrates. Large or repeated displacement may lead to peeling of the substrate.

In contrast, since the above configuration has a first substrate that is thicker than the remaining substrate and that has a side surface extending outside farther than that of the remaining substrate, the probability of contact of a substrate gripping means with the first substrate is relatively increased; the substrate gripping means gripping the side of a microchip, such as an operator's fingers, microchip fixing material and tweezers. On the other side, the probability of contact of the substrate gripping means with the remaining substrate is relatively decreased. Decreasing relatively the probability of contact of the substrate gripping means with the remaining substrates makes it possible to reduce the chance of deforming the remaining substrate due to the contact with the substrate gripping means because the rigidity of the remaining substrate, which has a smaller thickness than the first substrate, is usually relatively small compared to the first substrate. Reducing the chance of deforming the remaining substrate makes it harder for the remaining substrate to peel off from the first substrate, thus suppressing the peeling of the bonded substrate.

In the above configuration, the first substrate, of which probability of contact with the substrate gripping means is relatively high, has a side surface having a gradient. Thus, even when the substrate gripping means is in contact with the side surface of the first substrate, the force applied to the side surface thereof is distributed by the gradient. Hence, the force that deforms the first substrate, such as buckling and compression, becomes smaller. As describe above, when the force that deforms the first substrate is smaller, the remaining substrate becomes less likely to peel off from the first substrate, thereby preventing the peeling of the bonded substrate.

The remaining substrate may be composed of only a second substrate and the second substrate may have a side surface having a gradient when one end of the bonded substrate is viewed from a direction parallel to the main surface. Even when the substrate gripping means is in contact with the side surface of the second substrate, the gradient allows part of the contact force to escape, thus the force of deforming the first substrate is decreased, thereby the remaining substrate becomes unlikely to peel off from the first substrate. Therefore, this configuration prevents the peeling of the bonded substrate.

The remaining substrate may be composed of the second substrate and a third substrate, the third substrate being bonded to the first substrate on one main surface of the third substrate 30 and being bonded to the second substrate on the other main surface thereof; and the second substrate may have a side surface having a gradient when one end of the bonded substrate is viewed from a direction parallel to the main surface. Even in the case of the bonded substrate having the third substrate sandwiched between the first substrate and the second substrate, the gradient of the second substrate allows part of the contact force to escape, thus the force for deforming the first substrate is decreased, thereby the remaining substrate becomes unlikely to peel off from the first substrate. Therefore, this configuration prevents the peeling of the bonded substrate.

The second substrate may have a side surface extending outside farther than a side surface of the third substrate when one end of the bonded substrate is viewed from a direction parallel to the main surface. This configuration allows the probability of the contact of the substrate gripping means with the third substrate to be decreased, thus reducing the chance of deforming the third substrate due to the contact. Therefore, this configuration allows the third substrate to be unlikely to peel off from the first substrate or the second substrate, preventing the peeling of the bonded substrate.

The main surface of the first substrate, the main surface thereof being bonded to the remaining substrate, may include an outermost portion of one side surface of the first substrate when one end of the bonded substrate is viewed from a direction parallel to the main surface. The main surface of the second substrate, the main surface thereof being closest to the first substrate may include an outermost portion of one side surface of the second substrate when one end of the bonded substrate is viewed from a direction parallel to the main surface. This configuration allows each of the gradients in the first substrate and the second substrate to be formed from the respective outermost portions of the first substrate and the second substrate toward the main surface that is not bonded. In addition, the orientation of the gradient allows part of the pressing force with which the substrate gripping means presses the bonded substrate to act toward the direction of bonding the substrates to each other by reaction force. Therefore, this configuration prevents the peeling of the bonded substrate.

The side surface of the first substrate may preferably have no step to the side surface of the remaining substrate when one end of the bonded substrate is viewed from a direction parallel to the main surface. Since no step is between the side surface of the first substrate and that of the remaining substrate, no pressing force with which the substrate gripping means presses the bonded substrate is concentrated locally. Therefore, this configuration prevents the peeling of the bonded substrate.

The first substrate may include a non-bonded area in each of the both ends of the first substrate when the longitudinal side surface of the bonded substrate is viewed from a direction parallel to the main surface, the non-bonded area being an area where the first substrate is not bonded to the remaining substrate. This configuration allows the probability of contact of the substrate gripping means with the remaining substrate to be relatively decreased at the both ends of the bonded substrate in the longitudinal direction, thereby preventing the peeling of the bonded substrate.

The first substrate may include a non-bonded area in each of the both ends of the first substrate when the transverse side surface of the bonded substrate is viewed from a direction parallel to the main surface, the non-bonded area being an area where the first substrate is not bonded to the remaining substrate. This configuration allows the probability of contact of the substrate gripping means with the remaining substrate to be relatively decreased at the both ends of the bonded substrate in the transverse direction, thereby preventing the peeling of the bonded substrate.

When one of corners of the bonded substrate is viewed from a direction orthogonal to the main surface of the bonded substrate, the corner of the remaining substrate may exhibit a chamfered shape and the corner of the first substrate is located from the center of gravity of the bonded substrate farther than the corner of the remaining substrate. This configuration allows the probability of contact of the substrate gripping means with the corner of the remaining substrate to be relatively decreased. In addition, since the corner of the remaining substrate has a chamfered shape, the pressing force due to the substrate gripping means is distributed, thus the remaining substrate is less likely to be deformed. Therefore, this configuration prevents the peeling of the corner where the peeling of the bonded substrate is especially likely to occur.

At least the one corner of the first substrate may exhibit an arc-shaped chamfered shape and the corner of at least one substrate constituting the remaining substrate may exhibit a line shape or a polyline shape when one of the corners of the bonded substrate is viewed from a direction orthogonal to the main surface of the bonded substrate. This configuration relatively reduces the probability of contact of the substrate gripping means with the corner of the remaining substrate. In addition, since the first substrate exhibits an arc-shaped chamfered shape, the pressing force due to the substrate gripping means is distributed, thus the remaining substrate is less likely to be deformed. Therefore, this configuration prevents the peeling of the corner where the peeling of the bonded substrate is especially likely to occur.

### ADVANTAGE EFFECTS OF THE INVENTION

The present invention is capable of providing a microchip that reduces the risk of liquid leakage by effectively preventing the peeling of the bonded substrate.

### BRIEF DESCRIPTION OF DRAWINGS

FIG. 1 is a perspective view schematically illustrating a first embodiment of the microchip according to the present invention.
FIG. 2A is a view of an example of one end of a bonded substrate according to the first embodiment.
FIG. 2B is an enlarged view of area B in FIG. 2A.
FIG. 2C is an enlarged view of area C in FIG. 2A.
FIG. 3A is a view of one end of the bonded substrate according to a first modification example of the first embodiment.
FIG. 3B is a view of one end of the bonded substrate according to a second modification example of the first embodiment.
FIG. 3C is a view of one end of the bonded substrate according to a third modification example of the first embodiment.
FIG. 4A is a view of an example of the bonded substrate according to the first embodiment.
FIG. 4B is a view of another example of the bonded substrate according to the first embodiment.
FIG. 4C is a view of yet another example of the bonded substrate according to the first embodiment.
FIG. 5 is a view of one end of the bonded substrate according to a fourth modification example of the first embodiment.
FIG. 6 is a view of one end of a bonded substrate according to a second embodiment.
FIG. 7A is a view of one end of the bonded substrate according to a first modification example of the second embodiment.
FIG. 7B is a view of one end of the bonded substrate according to a second modification example of the second embodiment.
FIG. 8A is a view of one end of the bonded substrate according to a third modification example of the second embodiment.
FIG. 8B is a view of one end of the bonded substrate according to a fourth modification example of the second embodiment.
FIG. 9A is a view of one end of a bonded substrate according to a third embodiment.
FIG. 9B is a view of one end of the bonded substrate according to a first modification example of the third embodiment.
FIG. 9C is a view of one end of the bonded substrate according to a second modification example of the third embodiment.
FIG. 9D is a view of one end of the bonded substrate according to a third modification example of the third embodiment.
FIG. 10A is an enlarged view of a corner of the bonded substrate according to a fourth embodiment.
FIG. 10B is an enlarged view of a corner of the bonded substrate according to a first modification example of the fourth embodiment.
FIG. 11 is an enlarged view of a corner of the bonded substrate according to a second modification example of the fourth embodiment.

### DESCRIPTION OF EMBODIMENTS

A microchip according to the present invention will now be described with reference to the drawings. It is noted that each drawing disclosed in the present specification is merely schematically illustrated. In other words, the dimensional ratios on the drawings do not necessarily match the actual dimensional ratios, and the dimensional ratios between each drawing do not necessarily match either.

### [First Embodiment]

An example of a microchip accordingto the present invention will be described as a first embodiment.

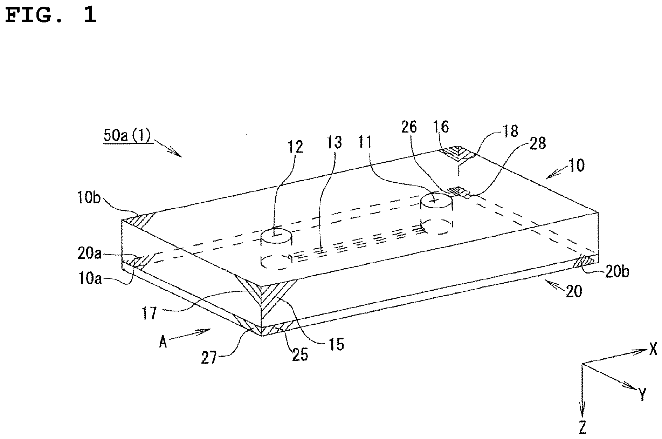

FIG. 1 is a perspective view schematically illustrating the present embodiment of a microchip. A microchip 1 includes a bonded substrate 50a that includes a first substrate 10 having a main surface 10a and a second substrate 20 having a main surface 20b, the main surface 10a of the first substrate 10 being stacked on the main surface 20a of the second substrate 20, and the two substrates being bonded together. The main surface refers to a surface that has a much larger area than the other surfaces constituting the substrate (10, 20). The main surface is not necessarily a flat surface, but may include an uneven surface, for example, with a groove. The first substrate 10 has two main surfaces (10a, 10b) and these two main surfaces are placed opposite each other. The second substrate 20 has two main surfaces (20a, 20b) and these two main surfaces are placed opposite each other. The bonded substrate 50a after being bonded has the main surface 10b of the first substrate 10 and the main surface 20b of the second substrate 20, both of the main surfaces being arranged to face each other.

Hereinafter, the bonded substrate 50a is described appropriately using the XYZ coordinate system such that the XY plane denotes the plane parallel to the main surfaces (10b, 20b) of the bonded substrate 50 and the Z direction denotes the direction orthogonal to the XY plane. With reference to the coordinate, the Z direction corresponds to the thickness direction of the bonded substrate 50a.

In the present specification, when the direction is expressed, a positive or negative sign is assigned to distinguish a positive direction from a negative direction, such as "+X direction" and "-X direction". In the case of expressing the direction without distinguishing a positive direction from a negative direction, it is simply expressed as "X direction". In other words, in the present specification, the simple expression of "X direction" includes both of "+X direction" and "-X direction". This also applies to the Y direction and the Z direction.

Other surfaces except for the main surfaces of the bonded substrate 50a are side surfaces. In the present embodiment, the first substrate 10 has two side surfaces (15, 16) that are substantially parallel to the XZ plane and have the X-axis direction as their longitudinal direction, and two side surfaces (17, 18) that are substantially parallel to the YZ plane and have the Y-axis direction as their longitudinal direction. The second substrate 20 similarly has two side surfaces (25, 26) that are substantially parallel to the XZ plane and have the X-axis direction as their longitudinal direction, and two side surfaces (27, 28) that are substantially parallel to the YZ plane and have the Y-axis direction as their longitudinal direction. The side surfaces will be discussed in detail later.

The substrates (10, 20) are preferably made of materials that are substantially non-porous body. Here, the term "substantially non-porous body" refers to a state that the apparent surface area of the substrates approximates to the actual surface area thereof. Examples of the materials that form the non-porous body as described above include inorganic materials such as glass and silicon; and resin materials such as polymethyl methacrylate (PMMA), poly carbonate (PC), cyclo-olefin copolymer (COC), cyclo-olefin polymer (COP), polystyrene (PS), and silicone. Furthermore, two or more of these resin materials can be combined. It is also possible to use different materials for the first substrate 10 and the second substrate 20.

The first substrate 10 has a groove on its main surface 10a. The main surface 10a is bonded to the main surface 20a of the second substrate 20. Upon the bonding, the area of the main surface 10a where no groove has been formed is bonded to the main surface 20a. The area of the main surface 10a where the groove has been formed is not bonded to the main surface 20a; the main surfaces (10a, 20a) are placed opposite each other in a way that the groove is covered with the second substrate 20 that acts on a lid. The groove with the lid placed thereon forms a hollow-shaped space. A part of the hollow-shaped space serves as a channel 13. The channel 13 is commonly referred to as a microchannel because of its minute width and other dimensions. However, the width and other dimensions are not limited to a micron size.

The channel 13 is provided with liquid distribution ports (11, 12) at both ends thereof, each port being open toward the main surface 10b of the first substrate 10. In the present embodiment, the second substrate 20 does not have grooves for forming channels or openings for serving as liquid distribution ports; however, the second substrate 20 may have grooves for forming channels and openings for serving as liquid distribution ports.

Various methods are available to provide grooves and openings in the substrates (10, 20), for example, injection molding, combination of photolithography process and etching process, casting, and machining process; however, the method can be appropriately selected according to the material constituting the substrates. For example, when the second substrate 20 is made of a resin material as described above, such as polymethyl methacrylate (PMMA), polycarbonate (PC), cyclo-olefin copolymer (COC), cyclo-olefin polymer (COP), polystyrene (PS) and silicone, injection molding is used to easily form a recess (13). The first substrate 10 may be made of a glass material such as borosilicate glass in addition to the above resin materials, if no recess is provided.

The liquid distribution ports (11, 12) are used as openings from which a liquid sample containing cells and tissues is injected or extracted. The liquid distribution ports (11, 12) has at least one of the following purposes: to inject a liquid sample into the microchip 1, and to discharge a liquid sample from the microchip 1. For example, the liquid distribution port 11 may be used as a liquid injection port and the liquid distribution port 12 may be used as a liquid discharge port. The state of the channel 13 is not limited to a state in which liquid flows when the liquid is in the channel 13. For example, the state includes a state with no liquid flow, such as a state in which liquid is stored. The shapes of the channel and the number of liquid distribution ports and their layout described above are examples, and are preferably designed to be desired.

The channel 13 is used for a place of culturing, mixing, separating, reacting, synthesizing or analyzing a liquid sample. When the place needs a large space to accommodate a liquid sample, a liquid sample reservoir for the liquid sample may be provided in the middle of the channel 13. In addition, the liquid sample reservoir may be used as a detection unit for fluorescence microscopy when the floor or wall of the large space immobilizes a substance that reacts with the object to be detected (e.g., a specific protein) to emit fluorescence.

### <Shape and dimension on the first substrate and the second substrate>

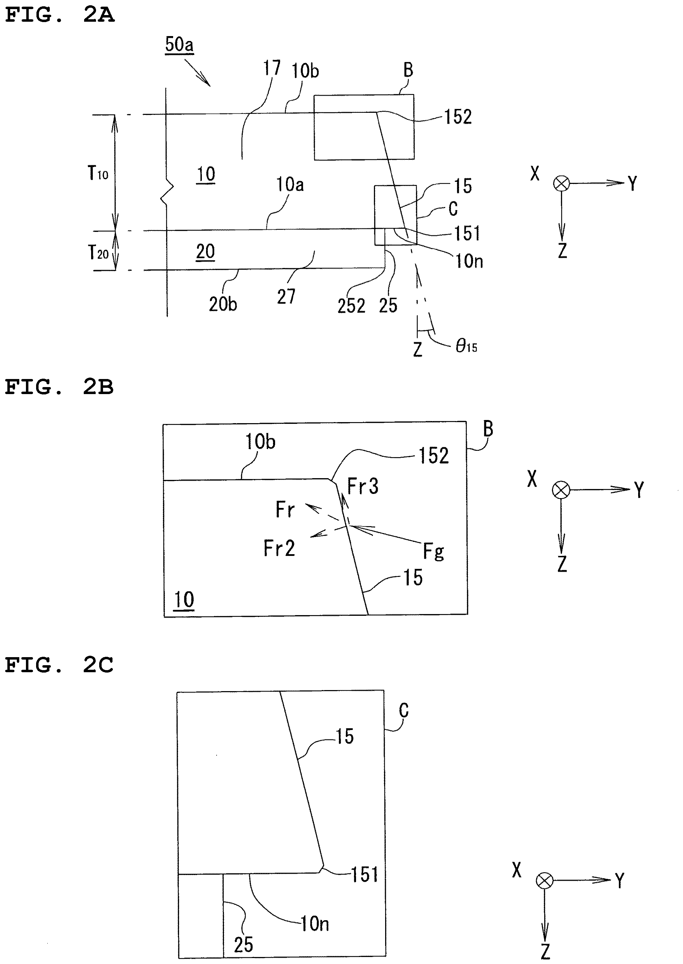

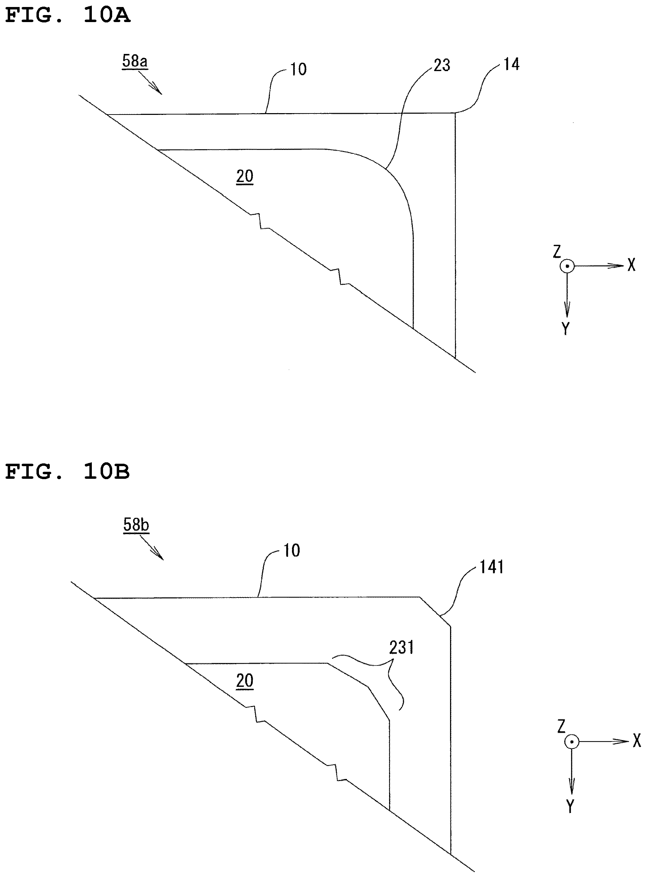

In the present embodiment, each of the first substrate 10 and the second substrate 20 is a substrate having a rectangular main surface. However, the main surfaces of the first substrate 10 and the second substrate 20 do not necessarily need to be rectangular. The main surfaces of the first substrate 10 and the second substrate 20 may be a square shape, a triangular shape, a polygonal shape with five or more corners, a circular shape or an oval shape. As will be described later in the fourth embodiment with reference to FIGS. 10A through 11, the corners of at least one of the first substrate 10 and the second substrate 20 may be chamfered in the first substrate 10 or the second substrate 20 having a polygonal main surface including a rectangular one. Hereinafter, the structure of each substrate (10, 20) will be described in detail with reference to the drawings in FIGS. 2A and below.

FIG. 2A is a side view of one end of the bonded substrate 50a viewed from the A arrow direction in FIG. 1. The A arrow direction is the direction parallel to the main surface of the bonded substrate 50a, which is the X direction in this case. The thickness T₁₀ of the first substrate 10 (width dimension in the Z direction) is larger than the thickness T₂₀ of the second substrate 20. The thickness T₁₀ of the first substrate 10 may be preferably 1 mm or more and 5 mm or less. The thickness T₁₀ of the first substrate 10 provides enough rigidity to prevent the microchip 1 from being bent in normal use.

The thickness T₂₀ of the second substrate 20 may be preferably 10 µm or more and 3 mm or less. The second substrate 20 is usually less rigid than the first substrate 10. For being used for observation and analysis, the microchip 1 has better observation and analysis performance when the thickness T₂₀ of the second substrate 20 becomes thinner. The second substrate 20 may be, for example, a flexible film-shaped substrate. Dimensions other than the thickness of the first substrate 10 and the second substrate 20 will be described later.

### (Side surface of the bonded substrate)

The microchip 1 is usually transported, positioned or fixed by gripping the side surface of the bonded substrate 50a. In other words, a substrate gripping means, such as the hand of a worker carrying the microchip 1 or a member for positioning or fixing the microchip 1, is contacted with the side surface of the bonded substrate 50a and applies a pressing force to the side surface thereof, the pressing force being large enough to support or fix the substrate 50a.

When the substrate gripping means is in contact with the side surface of the bonded substrate 50a, if the thickness T₁₀ of the first substrate 10 is larger than the thickness T₂₀ of the second substrate 20, the probability of contact with the first substrate 10 is relatively larger whereas the probability of contact with the second substrate 20 becomes relatively smaller. Since the second substrate 20 is usually less rigid than the first substrate 10, reducing the probability of contact with the second substrate 20, which is less rigid, lowers the risk of deforming the second substrate 20. Therefore, this configuration prevents the second substrate 20 from peeling off the first substrate 10.

In FIG. 2A, the side surface 17, which is substantially parallel to the YZ plane, is shown as a plane whereas the side surface 15, which is substantially parallel to the XZ plane, is shown as a line. As is similar for the second substrate 20, the side surface 27, which is substantially parallel to the YZ plane, is shown as a plane whereas the side surface 25, which is substantially parallel to the XZ plane, is shown as a line. In the case of the present embodiment, in which the bonded substrate 50a is rectangular, the entire side surfaces (15, 25) are recognized as one end. However, if the bonded substrate 50a is, for example, a circular substrate, only a partial area of the curved side surface is recognized as one end.

In FIG. 2A, the side surface 15, which is substantially parallel to the XZ plane, has a gradient when viewed in detail. Here, the first substrate 10 has a gradient such that the main surface 10a on the +Z side of the first substrate 10 (the side of the second substrate 20) is located outside farther than the main surface 10b on the -Z side thereof (the opposite side of the second substrate 20) when viewed in the Z direction. In short, the first substrate 10 has a gradient such that its length in the Y direction increases with approaching from the main surface 10b to the main surface 10a in the Z direction.

FIG. 2B is an enlarged view of the part B in FIG. 2A. This figure is used to describe the advantage of the side surface 15 having a gradient. The substrate gripping means is considered to apply the pressing force Fg necessary to support or fix the bonded substrate on the side surface 15 of the first substrate 10. The pressing force Fg provides a force Fr to the first substrate 10. The force Fr is decomposed into a force Fr2 acting perpendicular to the side surface 15 and a force Fr3 acting parallel to the side surface 15. While Fr2 is a factor that deforms the first substrate 10, Fr3 is not a factor that deforms the first substrate 10 because Fr3 is applied in the gradient direction. When Fr3 applied is greater than the frictional force, the gradient allows the substrate to slide from the substrate gripping means in the direction of escaping from the pressurized area. Accordingly, it will inevitably reduce the pressing force Fg of the substrate gripping means. (When the substrate is held by hand, force is relaxed to grip the substrate.) Hence, it is possible to reduce the force with which the substrate gripping means deforms the substrate. Therefore, this configuration allows the second substrate 20 to be unlikely to peel off from the first substrate 10.

In FIG. 2B, the corner 152 between the main surface 10b and the side surface 15 of the first substrate 10 exhibits a chamfered shape. FIG. 2C is an enlarged view of part C in FIG. 2A. In FIG. 2C, the corner 151 between the main surface 10a and the side surface 15 of the first substrate 10 exhibits a chamfered shape. In the present specification, the chamfered shape means a shape of seemingly trimming a corner and creating a new surface in the trimmed portion; thus the chamfer is not necessarily the actual trimming of the corner to create the chamfer. The substrate may be molded with a chamfered shape in the molding stage. Various types of chamfered shapes can be used, such as those with a flat surface including a C chamfer, and those with a curved surface including an R chamfer.

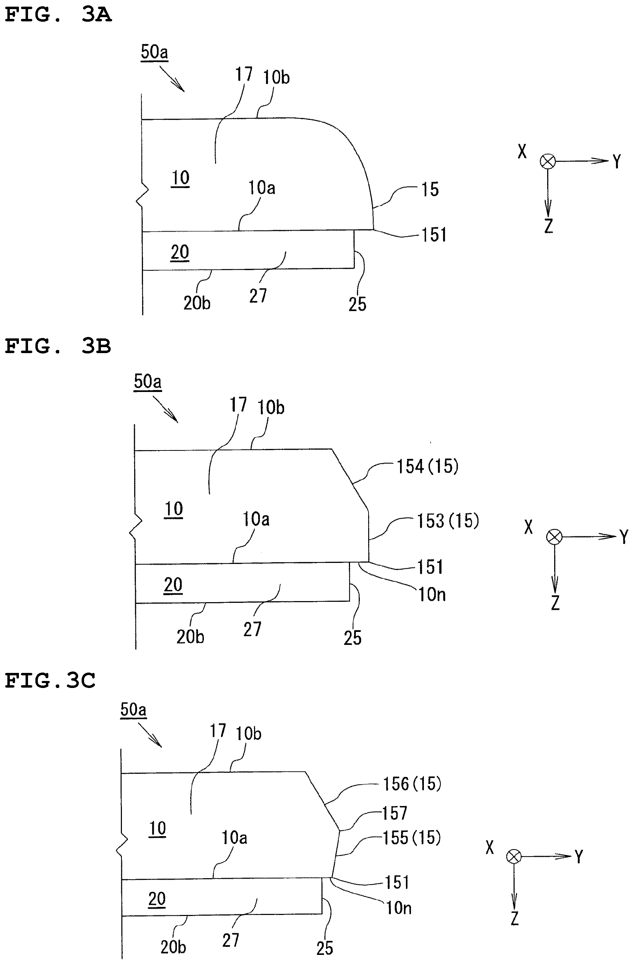

In the present specification, a chamfered shape provided between the main surface and the side surface refers to a new chamfered surface formed between the main surface and the side surface having a width of 1 mm or less in both of the Y direction and Z direction. When either one of the widths in the Y direction and Z direction exceeds 1 mm, it is considered to be a partial gradient. (This will be described in detail with reference to FIGS. 3B and 3C.)

Making the corners into a chamfered shape enables the pressing force of the substrate gripping means to be distributed. The distributed pressing force suppresses the occurrence of defects at the corners and prevents problems such as scattering of fragments caused by defects. In addition, it also reduces damage to other parts and injury to workers during handling. The description of the chamfer of any corner formed between the main surface and the side surface can be applied to any corner that the substrate has in the subsequent modifications and other embodiments.

In FIG. 2A, among the side surface 15 of the first substrate, the outermost portion thereof, which is the outermost portion (with the largest absolute value in the Y direction) when viewed in the Z direction, is a corner located on the main surface 10a of the first substrate 10. When the corner 151 exhibits a chamfered shape, the new surface created by the chamfer (e.g., the chamfered surface of the corner 151 in FIG. 2C) is considered as part constituting the main surface. Hence, even when the corner 151 exhibits a chamfered shape, the outermost portion is the corner located on the main surface 10a of the first substrate 10.

FIG. 2A shows that only one end of the bonded substrate 50a has a gradient having a predetermined angle with the Z direction; however, the other ends may have a predetermined angle with the Z direction. In other words, a side surface 16, a side surface 17 and a side surface 18 may be provided with a gradient similar to that of the side surface 15.

The gradient angle θ₁₅ (the angle between the direction along the side surface 15 and the thickness direction Z of the substrate) of the side surface 15 is preferably 1 degree or more, and is more preferably 3 degrees or more. This configuration effectively suppresses the force that causes deformation of the first substrate 10. In addition, the gradient angle θ₁₅ is preferably 5 degrees or less. This allows the pressing force by the gripping to be applied appropriately to the side surface 15 for supporting the bonded substrate in a stable manner.

The side surface 17, which is substantially parallel to the YZ plane of the first substrate 10, extends to the outermost portion of the bonded substrate 50a in the +Y direction. Then, the first substrate 10 in the present embodiment has a non-bonded area 10n on the outside (+Y side) of the second substrate 20 when viewed in the X direction or -Z direction. This configuration allows the probability of contact of the substrate gripping means with the side surface 15 of the first substrate 10 to be higher than the probability of contact of the substrate gripping means with the side surface 25 of the second substrate 20 when the substrate gripping means is in contact with the side surface of the bonded substrate 50a. As described above, since the second substrate 20 usually is relatively less rigid than the first substrate 10, this configuration reduces the chance of deforming the second substrate 20 due to contact. Therefore, the second substrate 20 becomes unlikely to peel off from the first substrate 10.

FIG. 2A shows that the side surface 17 extends to the outermost side of the bonded substrate 50a at one end of the substrate 50a and has a non-bonded area 10n; however, the side surface 17 may also extend to the outermost side of the bonded substrate 50a at the other end of the substrate 50a and has a non-bonded area 10n.

In FIG. 2A, the side surface 15 is composed of a plane with a constant gradient angle θ₁₅; however, the side surface 15 can take various forms. In the first modification example shown in FIG. 3A, the side surface 15 of the first substrate 10 is composed of a curved surface whose gradient angle θ₁₅ varies with the position in the thickness direction. In this figure, the side surface 15 is represented by a curve made up of multiple arcs. In the second modification shown in FIG. 3B, the side surface 15 of the first substrate 10 is composed of a partial gradient forming surface 154 and a plane 153 parallel to the XZ plane. In the third modification example shown in FIG. 3C, the side surface 15 is composed of two types of gradient forming surfaces (155, 156), and the intersection of the gradient forming surface 155 and the gradient forming surface 156 forms a outermost portion 157. As shown in FIGS. 3B and 3C, the gradient forming surfaces of the side surface, which have a partial side gradient, refer to a surface having at least either of the widths in the Y direction or the Z direction of the gradient surface exceeding 1 mm. In FIGS. 3B and 3C, the gradient angle in the partial gradient forming surface is not necessarily constant. The gradient angle on the partial gradient forming surface may vary depending on the position in the thickness direction.

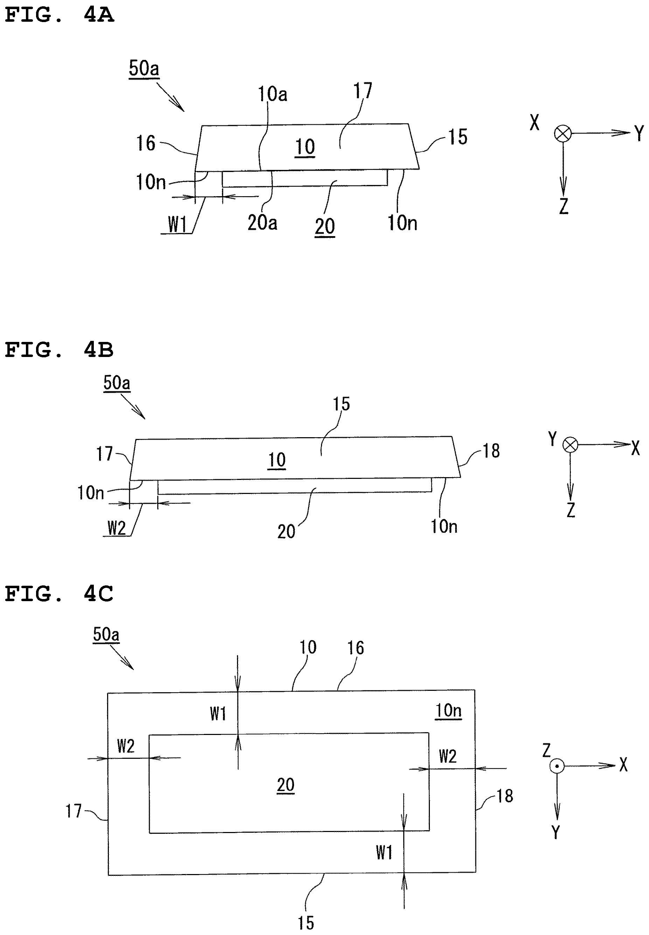

FIG. 4A shows an example of a bonded substrate having a side surface extending to the outermost side at a plurality of one ends and having a plurality of non-bonded areas. In FIG. 4A, the side surface 17, which is substantially parallel to the YZ plane, extends to the outermost side in +Y direction and -Y direction. On both ends of the first substrate 10 in the transverse direction (Y-axis direction), the first substrate 10 has non-bonded areas 10n where the first substrate 10 is not bonded to the second substrate 20.

It is preferable that the shortest distance between one end of the main surface 10a of the first substrate 10 in the transverse direction and one end of the main surface 20a of the second substrate 20 in the transverse direction, that is, a width W1 of the non-bonded area 10n in the transverse direction is 0.05 mm or more. This configuration makes it hard to make contact with the second substrate 20 at both ends of the bonded substrate 50a in the transverse direction. The width W1 may be preferably 0.5 mm or less to ensure the space for forming microchannels.

FIG. 4B shows another example of a bonded substrate having a side surface extending to the outermost side at a plurality of one ends and having a plurality of non-bonded areas. In FIG. 4B, the side surface 15, which is substantially parallel to the XZ plane, extends to the outermost side in +X direction and -X direction. On both ends of the first substrate 10 in the longitudinal direction (X-axis direction), the first substrate 10 has non-bonded areas 10n where the first substrate 10 is not bonded to the second substrate 20.

It is preferable that the shortest distance between one end of the main surface 10a of the second substrate 20 in the longitudinal direction and one end of the main surface 20a of the second substrate 20, the main surface 20a being bonded to the first substrate 10, in the longitudinal direction, that is, a width W2 of the non-bonded area 10n in the longitudinal direction is 0.05 mm or more. This configuration makes it hard to make contact with the second substrate 20 at both ends of the bonded substrate 50a in the longitudinal direction. The width W2 may be preferably 0.5 mm or less to ensure the space for forming microchannels.

FIG. 4C shows yet another example of a bonded substrate having a side surface of the first substrate 10 extending to the outermost side among four side surfaces enclosing the bonded substrate and having a plurality of non-bonded areas when the bonded substrate is viewed from the +Z direction. In the present embodiment, the bonded substrate 50a has non-bonded areas 10n with width W1 at both ends of the bonded substrate 50a in the longitudinal direction, and has non-bonded areas 10n of width W2 at both ends of the bonded substrate 50b in the longitudinal direction. The width W1 and width W2 can be the same or different values. Since the non-bonded areas 10n are included at each of both ends in the longitudinal and transverse directions, the probability of contact with the second substrate 20 is reduced even when the substrate gripping means is in contact from any direction.

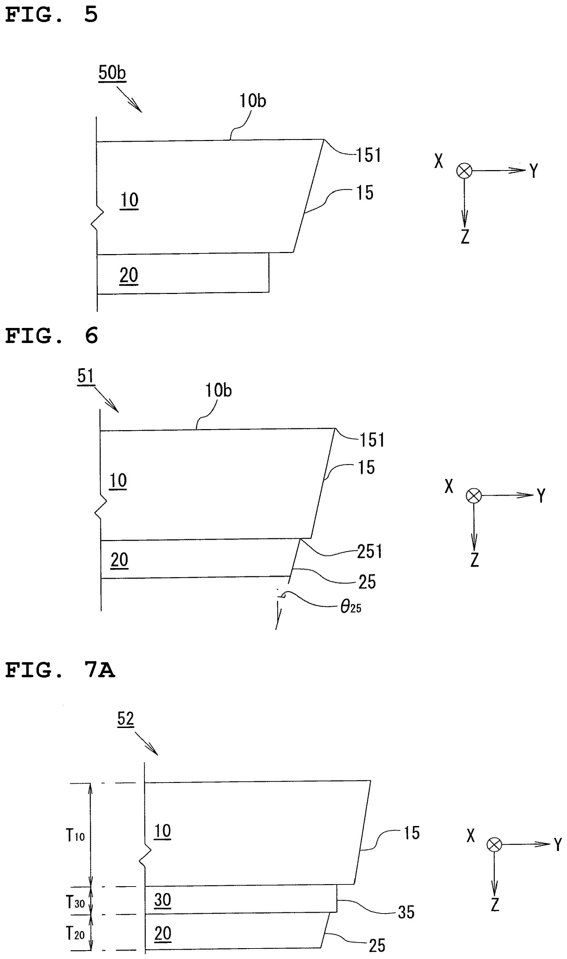

FIG. 5 is a fourth modification example of the first embodiment and shows one end of a bonded substrate 50b viewed from a direction parallel to the main surface of the first substrate 10 or the second substrate 20 (here in the X direction), as in FIG. 2A. Viewed from the Z direction, the corner 151, which is located at the outermost side in the side surface 15 of the first substrate 10, is located on the main surface 10b of the first substrate 10. Hence, the direction of the gradient of the first substrate 10 is opposite to that in FIG. 2A. Even with the orientation of the gradient shown in FIG. 5, the gradient allows part of the contact force to escape, thus suppressing the force that causes deformation of the first substrate 10. Therefore, this configuration allows the second substrate 20 to be unlikely to peeling off from the first substrate 10.

The corner 151 in FIG. 5 forms an acute angle. When the corner 151 is chamfered, the above-mentioned effects related to the chamfered shape, such as suppressing the occurrence of defects, can be obtained. However, a small defect in the vicinity of the corner 151 usually does not reach the microchannel located internally and thus causes no liquid leakage problem. Furthermore, this acute corner allows part of the sudden excessive force to be absorbed by the occurrence of defect, and suppresses the force that causes deformation in the first substrate 10, hence the corner 151 may be left at an acute angle without being chamfered. The same applies to the subsequent embodiments and variations in which the respective substrates have acute corners.

### <Method of bonding substrates>

When the first substrate 10 and the second substrate 20 are to be bonded together, adhesives or adhesive tapes may be used; however, in the present embodiment, the following processes that require no adhesives or other materials are used for bonding them.

First, a surface activation treatment is performed on the bonding surfaces (10a, 20a) of both substrates. Methods of the surface activation treatment include the irradiation with ultraviolet light or the contact with plasma gas.

The method of ultraviolet irradiation is performed by irradiating the main surface 20a of the second substrate 20 and the main surface 10a of the first substrate 10 with vacuum ultraviolet light, which has a wavelength of 200nm or less, emitting from an ultraviolet light source, for example, a xenon excimer lamp having an emission line at a wavelength of 172 nm. Other examples of ultraviolet light sources include low-pressure mercury lamps having an emission line at a wavelength of 185 nm and deuterium lamps having an emission line in a wavelength range of 120 to 200 nm. The irradiance of the vacuum ultraviolet light is, for example, 5 to 500 mW/cm², and the irradiation time is appropriately set according to the resin; however, it is, for example, 5 to 600 seconds.

The method of contacting with the plasma gas is performed by contacting the main surface 20a of the second substrate 20 and the main surface 10a of the first substrate 10 with substance into which process gas is transformed with atmospheric pressure plasma. The process gas may be composed of mainly nitrogen gas or argon gas with oxygen gas of 0.01 to 5% by volume. A mixture gas of nitrogen gas and clean dry air (CDA) can also be used. The contact time of the plasma gas is, for example, 5 to 100 seconds.

Next, the second substrate 20 is overlaid on the first substrate 10 so as to be in contact with both of the bonding surfaces (10a, 20a), on which the surface activation treatment have been performed. The two substrates are pressed using press machines to perform a bonding process. In order to maintain the surface activation state, the bonding process may be preferably performed within a predetermined time, for example, within ten minutes, after the completion of the ultraviolet irradiation process.

This bonding process is performed under heated conditions, if necessary, to reinforce the bonding strength. In the bonding process, the bonding conditions such as heating temperatures and pressing forces are set according to the constituent material in the first substrate 10 and the constituent material in the second substrate 20. Examples of the specific conditions include that the temperature during the pressing is 40 to 150°C and the pressing force for the bonding is 0.1 to 10 MPa.

The substrate, which has been formed by bonding the first substrate 10 with the second substrate 20, may be heated further at a predetermined time as necessary after being pressurized at a predetermined time. Even when the substrate, which is stacked with pressurization, has a mixture of an area with a sufficient bonding state and an area with an insufficient bonding state at its bonding interface, heating the bonded substrate 50a after the pressurization enables the area with the insufficient bonding state to have a desired state.

After through the cooling process, the bonded substrate 50a, in which the second substrate 20 has been bonded onto one main surface of the first substrate 10, is fabricated.

### [Second Embodiment]

The second embodiment is the same as the first embodiment except the shape of the bonded substrate of the microchip configured as described below, thus common items will be omitted. The same applies to the third embodiment.

FIG. 6 shows the bonded substrate 51 of a microchip of the second embodiment. One end of the bonded substrate 51 viewed from the X direction is shown as similar to FIG. 2A. In the present embodiment, the side surface 25 of the second substrate 20 also has a gradient in addition to the side surface 15 of the first substrate 10.

The probability of contact of the substrate gripping means with the side surface 25 of the second substrate 20 is lower than the probability of contact with the side 15 of the first substrate 10; however, it is not zero. Even when the substrate gripping means is in contact with the side surface 25, the gradient of the side surface 25 allows part of the contact force to escape, hence the force that causes deformation of the second substrate 20 as a whole becomes smaller, making it less likely for the second substrate 20 to peel off from the first substrate 10.

The gradient angle θ₂₅ (the angle between the direction along the side surface 25 and the thickness direction Z of the substrate) of the side surface 25 is preferably 1 degree or more, and is more preferably 3 degrees or more. This configuration effectively suppress the force that causes deformation of the second substrate 20. In addition, the gradient angle θ₂₅ is preferably 5 degrees or less. This allows the pressing force by the gripping to be applied appropriately to the side surface 25 for supporting the bonded substrate in a stable manner. The side surface 26, side surface 27 and side surface 28 may also have gradients similar to side surface 25.

FIG. 7A is a first modification example of the second embodiment. The first modification example includes a bonded substrate 52 composed of a first substrate 10, a second substrate 20, and a third substrate 30 that is bonded to the first substrate 10 on one main surface of the third substrate 30 and is bonded to the second substrate 20 on the other main surface thereof. The figure shows one end of the bonded substrate 52 viewed from a direction parallel to the main surface of each substrate. In the bonded substrate 52, the thickness T₃₀ of the third substrate 30 is smaller than the thickness T₁₀ of the first substrate 10. Furthermore, the thickness T₃₀ of the third substrate may be smaller than the thickness T₂₀ of the second substrate. The third substrate 30 may be, for example, a porous membrane through which certain cells and tissues can pass. The use of a porous membrane for the third substrate 30 enables the microchip composed of the bonded substrate 52 to be used for cell and tissue separation and other applications.

The side surface 15 of the first substrate 10 is located outside farther than the side surface 35 of the third substrate 30 when the bonded substrate 52 is viewed from the Z direction. When the area illustrated in FIG. 7A is focused on, the side surface 15 of the first substrate 10 is located on the +Y side compared with the side 35 of the third substrate. This configuration relatively increases the probability of contact of the substrate gripping means with the side surface 15, whereas relatively reduces the probability of contact of the substrate gripping means with the side surface 35 of the third substrate 30, which is thinner than the first substrate 10. In the present embodiment, the side surface 15 of the first substrate 10 and the side surface 25 of the second substrate 20 each have a gradient; however, the side surface 25 of the second substrate 20 may not have a gradient.

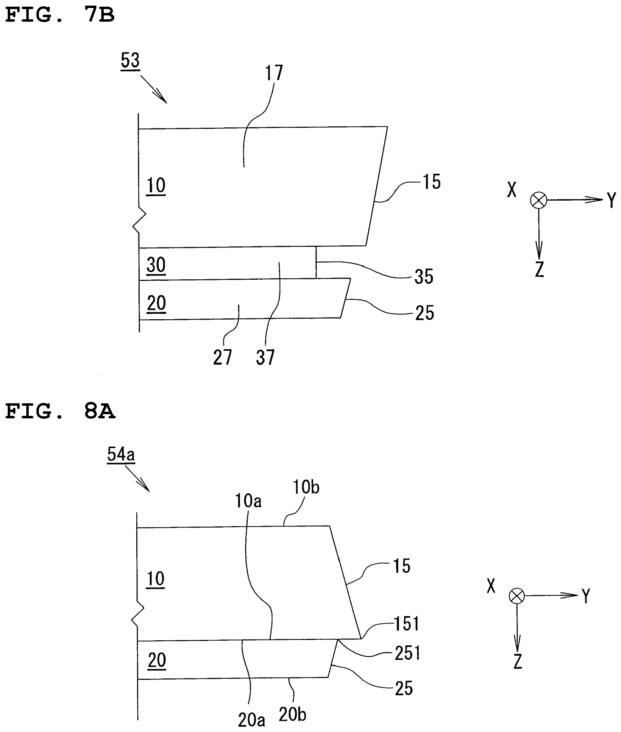

FIG. 7B is a second modification example of the second embodiment and shows one end of the bonded substrate 53 viewed from a direction parallel to a main surface of each substrate. In the present modification, the side surface 27 of the second substrate 20 extends outside farther than the side surface 37 of the third substrate 30 in addition to the side surface 17 of the first substrate 10. When the area illustrated in FIG. 7B is focused on, the side surface 15 of the first substrate 10 and the side surface 25 of the second substrate 20 are located outside (+Y side) farther than the side surface 35 of the third substrate 30 when viewed from the Z direction. This configuration relatively increases the probability of contact of the substrate gripping means with the side surface 15 and the side surface 25, whereas relatively reduces the probability of contact of the substrate gripping means with the side surface 35 of the third substrate 30.

FIG. 8A is a third modification example of the second embodiment and shows one end of a bonded substrate 54a viewed from a direction parallel to a main surface of each substrate as similar to the above embodiments. When the area illustrated in FIG. 8A is focused on, the corner 151 is located at the outermost side (+Y side) of the side surface 15 of the first substrate 10 when the bonded substrate 54a is viewed from the Z direction. In other word, the corner 151 is located away from the center of gravity of the first substrate 10 in the side surface 15 of the first substrate 10.

The main surface 10a of the first substrate 10 where the corner 151 is located is the surface that is bonded to the second substrate 20. When the area illustrated in FIG. 8A is focused on, the corner 251 is located at the outermost side (+Y side) of the side surface 25 of the second substrate 20. The main surface 20a of the second substrate 20 where the corner 251 is located is the surface that is bonded to the first substrate 10. In short, the main surfaces (10a, 20a) where the corners (151, 251) are located are inside the thickness direction of the bonded substrate compared with the opposing main surfaces (10b, 20b) in the respective substrates.

This configuration forms a gradient from the inner corners (151, 251) in the thickness direction to the outside of the respective substrates in the thickness direction. The orientation of the gradient allows part of the pressing force with which the substrate gripping means presses the bonded substrates 54a to apply in the direction of bonding the bonded substrates 54a to each other by reaction force. Therefore, this configuration prevents the second substrate 20 from peeling off the first substrate 10.

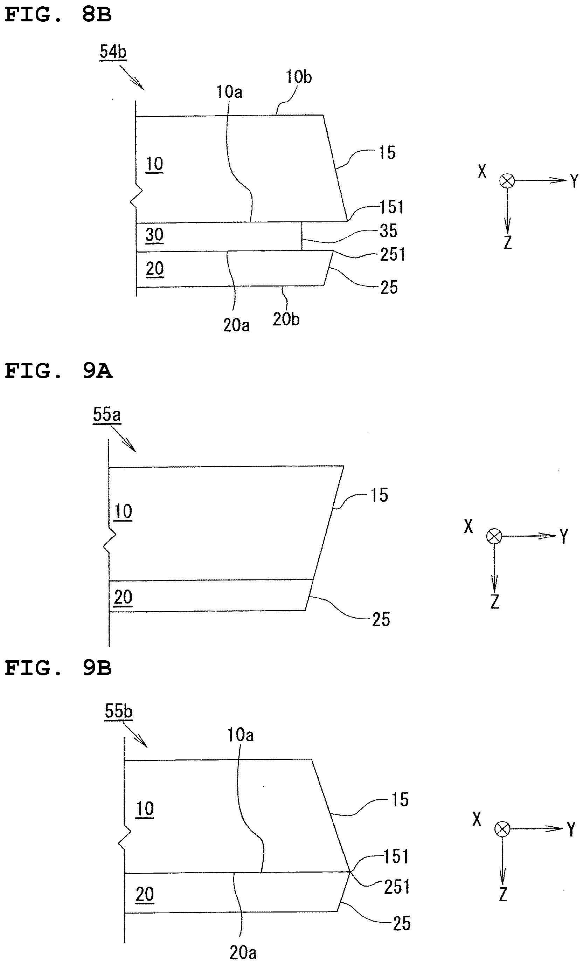

FIG. 8B is a fourth modification example of the second embodiment and shows one end of a bonded substrate 54b viewed from a direction parallel to the main surface of each substrate, as in the embodiment described above. The bonded substrate 54b includes a first substrate 10, a second substrate 20, and a third substrate 30 that is bonded to the first substrate 10 on one main surface of the third substrate 30 and is bonded to the second substrate 20 on the other main surface thereof.

When the area illustrated in FIG. 8B is focused on, the corner 151 of the first substrate 10 is located at the outermost side (+Y side) of the side surface 15 of the first substrate 10 when viewed from the Z direction. The main surface 10a of the first substrate 10 where the corner 151 is located is the surface that is bonded to one main surface of the third substrate 30 that constitutes the remaining substrate. The corner 251 is located at the outermost side (+Y side) of the side surface 25 of the second substrate 20 when viewed from the Z direction. In the second substrate 20, the main surface 20a, which is closest to the first substrate 10, includes the corner 251. In short, even in this modification, the main surfaces (10a, 20a) where the corners (151, 251) are located are inside the thickness direction of the bonded substrate compared with the opposing main surfaces (10b, 20b) in the respective substrates.

This configuration forms a gradient from the inner corners (151, 251) to the outside of the respective substrates in the thickness direction. In the present embodiment, the orientation of the gradient allows part of the pressing force with which the substrate gripping means presses the bonded substrates 54b to apply in the direction of bonding the bonded substrates 54b to each other by reaction force. Moreover, since both of the main surfaces (10a, 20a), on which the first substrate 10 and the second substrate 20 are in contact with the third substrate 30, contain corners (151, 251), the third substrate 30 is located deeply inside from the side surface of the first substrate 10 and the second substrate 20, thereby further reducing the probability of contact of the substrate gripping means with the side surface 35 of the third substrate 30.

### [Third embodiment]

FIG. 9A is a third embodiment of the microchip and shows one end of the bonded substrate 55a viewed from a direction parallel to a main surface of each substrate, as similar to the above-mentioned embodiment. The side surface 15 of the first substrate 10 is configured to have no step with the side surface 25 of the second substrate 20 that is bonded. This configuration prevents the pressing force with which the substrate gripping means presses the bonded substrate including its corners from being concentrated locally and allows the pressing force to be distributed on the entire side surfaces (15, 25) of the bonded substrate 55a. Hence, the force that causes deformation of the bonded substrate 55a as a whole becomes smaller, making it less likely for the second substrate 20 to peel off from the first substrate 10. Furthermore, bonding without the step (a non-bonded surface) enables the bonding area of the substrate to be increased, enhancing the bonding strength.

FIG. 9B is a first modification example of the third embodiment. Even in the first modification example, the side surface 15 of the first substrate 10 is configured to have no step with the side surface 25 of the second substrate 20 that is bonded. Bonding the main surface 10a including the corner 151 with the main surface 20a including the corner 251 maximizes the bonded area in the bonded substrate, enhancing the bonding strength. Moreover, the orientation of the gradient of both side surfaces (15, 25) allows part of the pressing force with which the substrate gripping means presses the bonded substrates 55b to apply in the direction of bonding the bonded substrates 55b to each other by reaction force, preventing peeling of the bonded substrate 55b.

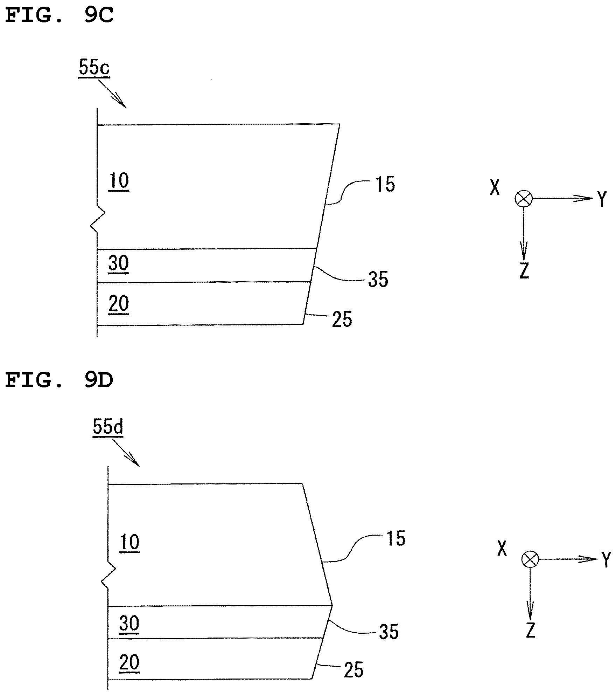

The similar effect as in FIGS. 9A and 9B, which are composed of two substrates, can also be obtained in the case of three substrates. FIG. 9C is a second modification example of the third embodiment. FIG. 9D is a third modification example of the third embodiment. Each of the bonded substrates (55c, 55d) includes a first substrate 10, a second substrate 20, and a third substrate 30 that is bonded to the first substrate 10 on one main surface of the third substrate 30 and is bonded to the second substrate 20 on the other main surface thereof. The side surface 15 of the first substrate 10 is configured to have no step with the side surface 35 of the third substrate 30 that is bonded. The side surface 25 of the second substrate 20 is configured to have no step with the side surface 35 of the third substrate 30 that is bonded.

### [Fourth Embodiment]

FIG. 10A is an enlarged view of one corner (the corner between two side surfaces of the substrate) of the microchip of the fourth embodiment when viewed from the +Z direction of the bonded substrate 58a. In other words, this figure shows one of the corners of the bonded substrate 58a, which includes the first substrate 10 and the second substrate 20, when viewed from the second substrate 20 side of the bonded substrate 58a. The corners of the second substrate 20 exhibit a chamfered shape 23, and the corners 14 of the first substrate 10 are located from the center of gravity of the bonded substrate 58a farther than the chamfered shape 23 of the second substrate. The chamfered shape means a shape of seemingly trimming a corner and creating a new surface in the trimmed portion; thus the chamfer is not necessarily the actual trimming of the corner to create the chamfer.

Such a corner between the two side surfaces of the substrate is particularly prone to peeling off due to the contact and pressure of the substrate gripping means. However, making the corner between the two side surfaces of the second substrate 20 into a chamfered shape 23 enables the probability of contact with the corner (chambered shape 23) to be relatively decreased. In addition, even when the substrate gripping means is in contact with the chamfered shape 23 of the second substrate, the pressing force due to the substrate gripping means is distributed because the contact of the substrate gripping means is received on the face, thus the second substrate 20 is less likely to be deformed.

In the present embodiment, the chambered shape 23 exhibits an R chamfer, i.e., a curved surface without forming a corner. However, the chamfered shape 23 may also be a C-chamfer, i.e., the two intersecting substrate side surfaces are chamfered to the same width (in other words, the chamfered portion is chamfered to form an isosceles triangle). An R chamfer and a C chamfer is highly effective in distributing the pressing force applied by the substrate gripping means. The two intersecting substrate side surfaces may also be chamfered at different widths from each other (the triangle in the chamfered portion is not necessarily isosceles). Furthermore, in the above embodiment, one corner of the bonded substrate 58a is explained to be chamfered; however, the other corners may be chamfered in the same way. The same applies to the modification examples described later.

FIG. 10B is an enlarged view of a corner of the bonded substrate 58b according to a first modification example of the fourth embodiment. The corner of the first substrate 10 has a C chamfer shape 141. The corner of the second substrate 20 has a chamfered portion in a polyline shape 231, i.e., a plurality of sides with different angles (two sides in FIG. 10B). By increasing the number of sides in the chamfered portion, the angle created in the chamfered portion increases, thus the pressing force by the substrate gripping means is distributed. Increasing the number of sides to infinity makes it an R-chamfered shape. All of the chamfered portions shown in FIGS. 10A and 10B are convex shapes projecting outward from the substrate; however, they can also be concave shapes (projecting inward into the substrate).

In the first to third embodiments, the first substrate 10 and the second substrate 20 are provided with a gradient on their sides in the thickness direction (the Z direction); however, these substrates (10, 20) may not be provided with a gradient on their sides in the thickness direction, instead may be provided with a gradient on only their corners. Providing the gradient at the corners of the substrate, where the substrate gripping means is more likely to be in contact with the substrate than at the sides of the substrate, prevents the deformation due to the pressing force applied by the substrate gripping means.

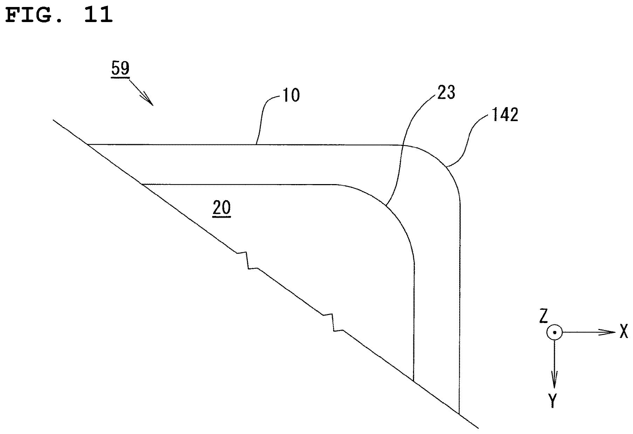

FIG. 11 is a second modification example of the fourth embodiment and an enlarged view of one corner of the bonded substrate 59 when viewed from the +Z direction. In other words, FIG. 11 shows the corner of the bonded substrate 59 composed of the first substrate 10 and the second substrate 20, when viewed from the second substrate 20 side. The corners of the first substrate 10 and the second substrate 20 each have an R-chamfered shape, i.e., an arc-shaped chamfered shape.

This configuration relatively reduces the probability of contact of the substrate gripping means with the corner of the chamfered shape 23 of the second substrate 20. In addition, since the corners of both of the substrates each exhibit an arc-shaped chamfered shape, the pressing force due to the substrate gripping means is distributed, thus the remaining substrate is less likely to be deformed. The corners of both of the substrates, however, may have a line shape or a polyline shape.

In the above embodiments and various modification examples, among the multiple substrates constituting the bonded substrate, the substrate with a relatively large thickness is referred to as the first substrate, and the substrate with a relatively small thickness is referred to as the second (and third) substrate. In other words, the first substrate is determined based on the thickness of the respective substrates. However, the first substrate may be determined based on the rigidity of the substrate. In other words, among the multiple substrates constituting the bonded substrate, the substrate with relatively high rigidity may be referred to as the first substrate, and the substrate with relatively low rigidity may be referred to as the remaining substrates (the second and third substrates). In all the above-mentioned descriptions, "substrate with relatively high rigidity" and "substrate with relatively low rigidity" can also be employed, instead of "substrate with a relatively large thickness" and "substrate with a relatively small thickness", respectively. The rigidity of the substrate is determined by the material of the substrate and the size, number and arrangement of the grooves and openings provided in the substrate. When substrates with different rigidity from each other are used, the substrates with different rigidity from each other may have the same thickness.

In the above embodiments, only bonded substrates composed of two substrates and bonded substrates composed of three substrates are disclosed; however, bonded substrates composed of four or more substrates may also be applicable. The above-mentioned embodiments or various modification examples may also be combined as appropriate. The present invention is not limited to the embodiments described above, and can be applicable to various improvements and modifications without departing from the scope of the present invention.

- 1: Microchip
- 10: First substrate
- 10a, 10b: Main surface (of first substrate)
- 10n: non-bonded area
- 11, 12: Liquid distribution port
- 13: Channel
- 14: Corner
- 15, 16, 17, 18: Side surface (of first substrate)
- 20: Second substrate
- 20a, 20b: Main surface (of second substrate)
- 23: Chamfered shape
- 25, 26, 27, 28: Side surface (of second substrate)
- 30: Third substrate
- 35, 37: Side surface (Third substrate)
- 50a, 50b, 51, 52, 53, 54a, 54b, 55a to 55d, 58a, 58b, 59:: Bonded substrate
- 141:: C chamfered shape
- 151, 152, 251:: Corner
- 154, 155, 156:: Gradient forming surface
- 157:: Outermost portion
- 231:: Polyline shape

## Claims

1. A microchip having a microchannel located inside a bonded substrate composed of a plurality of substrates, each of the substrates having a main surface and a side surface, the main surfaces being bonded each other,
wherein a plurality of the substrates are composed of a first substrate with a large thickness and a remaining substrate with a smaller thickness than the first substrate, and at least the first substrate has a side surface having a gradient and extending to an outermost side of the bonded substrate when one end of the bonded substrate is viewed from a direction parallel to the main surface.

2. The microchip according to claim 1, wherein the remaining substrate is composed of only a second substrate and the second substrate has a side surface having a gradient when one end of the bonded substrate is viewed from a direction parallel to the main surface.

3. The microchip according to claim 1, wherein the remaining substrate is composed of the second substrate and a third substrate, the third substrate being bonded to the first substrate on one main surface of the third substrate and being bonded to the second substrate on the other main surface thereof; and the second substrate has a side surface having a gradient when one end of the bonded substrate is viewed from a direction parallel to the main surface.

4. The microchip according to claim 3, wherein the second substrate has a side surface extending outside farther than a side surface of the third substrate when one end of the bonded substrate is viewed from a direction parallel to the main surface.

5. The microchip according to any one of claims 2 to 4, wherein the main surface of the first substrate, the main surface thereof being bonded to the remaining substrate, includes an outermost portion of one side surface of the first substrate when one end of the bonded substrate is viewed from a direction parallel to the main surface; and the main surface of the second substrate, the main surface thereof being closest to the first substrate includes an outermost portion of one side surface of the second substrate when one end of the bonded substrate is viewed from a direction parallel to the main surface.

6. The microchip according to any one of claims 2 to 5, wherein the side surface of the first substrate has no step to the side surface of the remaining substrate when one end of the bonded substrate is viewed from a direction parallel to the main surface.

7. The microchip according to any one of claims 1 to 5, wherein the first substrate includes a non-bonded area in each of the both ends of the first substrate when a longitudinal side surface of the bonded substrate is viewed from a direction parallel to the main surface, the non-bonded area being an area where the first substrate is not bonded to the remaining substrate.

8. The microchip according to any one of claims 1 to 5 or 7, wherein the first substrate includes a non-bonded area in each of the both ends of the first substrate when a transverse side surface of the bonded substrate is viewed from a direction parallel to the main surface, the non-bonded area being an area where the first substrate is not bonded to the remaining substrate.

9. The microchip according to any one of claims 1 to 8, wherein when one of corners of the bonded substrate is viewed from a direction orthogonal to the main surface of the bonded substrate, the corner of the remaining substrate exhibits a chamfered shape and the corner of the first substrate is located from the center of gravity of the bonded substrate farther than the corner of the remaining substrate.

10. The microchip according to claims 9, wherein at least the one corner of the first substrate exhibits an arc-shaped chamfered shape and the corner of at least one substrate constituting the remaining substrate exhibits a line shape or a polyline shape when one of the corners of the bonded substrate is viewed from a direction orthogonal to the main surface of the bonded substrate.
